# EUROPEAN PATENT SPECIFICATION

(11) **EP 3 419 585 B1**
(45) Date of publication and mention of the grant of the patent: **06.04.2022**
(21) Application number: 17706826.9
(22) Date of filing: 27.02.2017
(51) Int. Cl.: A61K 6/891

(54) **THERMOPLASTIC DENTURE FRAME, MANUFACTURE METHOD AND DENTURE**
THERMOPLASTISCHER PROSTHESENRAHMEN, HERSTELLUNGSVERFAHREN UND ZAHNERSATZ
CADRE DE DENTURE THERMOPLASTIQUE, PROCÉDÉ DE FABRICATION ET DENTURE

(30) Priority: 25.02.2016 US 201662299657 P; 30.05.2016 EP 16171913; 14.11.2016 US 201662421532 P
(43) Date of publication of application: 02.01.2019
(73) Proprietor: Myerson, LLC, Chicago, IL 60640 (US)
(72) Inventor: SHEMPER, Bianca Sadicoff, Hattiesburg, MS 39402 (US); SHARY, Timothy James, Atlanta, GA 30329 (US)
(74) Representative: Benvenuti, Federica
(86) International application number: PCT/EP2017/054531
(87) International publication number: WO 2017/144727

(56) References cited:
- EP-B1- 2 200 672
- GB-A- 2 523 004
- US-A1- 2014 322 462
- US-A1- 2015 011 673

## Description

### CROSS-REFERENCE TO RELATED APPLICATIONS

This application claims priority to U.S. Provisional Patent Application No. 62/299,657, filed February 25, 2016; U.S. Provisional Patent Application No. 62/421,532, filed November 14, 2016; and European Patent Application number EP 16171913.3, filed May 30, 2016, which claims priority to U.S. Provisional Patent Application No. 62/299,657, filed February 25, 2016.

### FIELD OF THE INVENTION

The present invention relates to denture frames include at least one poly(ether ether) ketone polymer and at least one polyphenylsulfone polymer. The invention further relates to methods of making a denture frame.

### BACKGROUND OF THE INVENTION

Dentures are designed to replace missing teeth. Dentures generally consist of a removable plate (or frame) that holds one or more teeth. Traditionally, dentures include a metal frame, which is desirable due to the stress resistance, durability, and stain resistance of the material. However, the use of metal has numerous disadvantages such as undesirable aesthetics, stiffness, and weight, as well as design and manufacturing limitations that can lead to poor fit and patient discomfort or dissatisfaction. Attempts have been made to address some of the deficiencies of metal by making dental prostheses from a thermoplastic polymer such as a poly(ether ether ketone) polymer; however a need remains for dental prostheses with improved toughness, flexibility, color stability and dimensional stability, leading to improved product lifetimes.

EP 2 200 672 discloses a composition comprising, among others, PEEK and poly(aryl ether) sultane. Said composition may include particulate fillers, TiO2 as pigment and other additives. The composition is used for manufacturing dental structures, such as dentures.

US 2015/0011673 discloses a composite material for use in the manufacture of dentures and/or denture bases. The material comprises a mixture of PEEK and PPSU and inorganic particles (1-10 microns diameter). The material is pre-processed by melt-kneading and a shape-farming process before being further processed by e.g. injection molding or machining.

US 2014/0322462 discloses another composite material for producing dental articles by e.g. injection/ extrusion molding. The material comprises 35% PEEK and 35% PPSU.

GB 2 523 004 discloses a method for making prosthodontics devices made of PEEK. Said method comprises a step of milling a blank comprising the PEEK composition.

### BRIEF DESCRIPTION OF DRAWINGS

Fig. 1 is a schematic depiction of a top-down view of a mandibular partial removable denture.
Fig. 2 is a schematic depiction of a top-down view of a mandibular partial removable denture frame.
Fig. 3 is a schematic depiction of a top-down view of the mandibular partial removable denture frame of Fig. 2 positioned in a patient's mouth.
Fig. 4 is a schematic depiction of a top-down view of a portion of a denture frame showing a finish line.
Fig. 5 is a schematic depiction of a perspective view of a region of a denture frame showing a finish line.
Fig. 6 is a schematic depiction of a cross section of a finish line.
Fig. 7A and 7B are a schematic depictions of a cross section of a portion of a denture displaying a finish line with a cupped inner surface and a portion of an artificial gum, where Fig. 7A depicts the denture without flex and Fig. 7B depicts the denture under flexing.
Fig. 8 is schematic depiction of a cross section of a portion of a denture frame showing a finish line having a symmetric cross section.

### DETAILED DESCRIPTION OF THE INVENTION

Described herein are thermoplastic denture frames having significantly improved lifetimes as well as comfort. The general lifetime of a denture frame (and denture) is a function of both its aesthetic characteristics and its mechanical characteristics. The denture frames described herein include a polymer compositions including at least one poly(ether ether ketone) ("PEEK") polymer and at least one polyphenylsulfone ("PPSU") polymer. Aesthetically, it was surprisingly discovered that the polymer compositions have significantly improved color stability, relative to corresponding polymer compositions the including the at least one PEEK polymer as the only polymeric component of the polymer composition. Mechanically, the polymer compositions additionally have significantly improved toughness, flexibility and dimensional stability. The combination of the aesthetic characteristics and mechanical characteristics of the polymer compositions allows for denture frames having improved lifetimes and comfort.

The general lifetime of a denture frame is a function of both its aesthetic characteristics and its mechanical characteristics. With respect to aesthetics, given the desire for concealed use, the denture frames described herein have significantly improved aesthetic characteristics relative to denture frames including a corresponding polymer compositions including the at least one PEEK polymer as the only polymer. While dentures provide a biomechanical benefit *(e.g.* increased chewing ability), the aesthetic nature of the denture significantly impacts its customer appeal. For example, denture frames that take on a more natural appearance in the the oral environment into which they are inserted are highly preferable, as they help to conceal the presence of the denture frame itself. Denture design elements such as color matching, frame thickness and fitment, among other characteristics, help to conceal the denture when placed in the oral cavity. However, the oral cavity is a chemically harsh environment. Some types of common foods and drinks *(e.g.* coffee and wine) can be harsh staining agents, which come into contact with the denture (and, of course, the denture frame) during their intended and normal course of use. Despite cleaning, dentures eventually stain to an extent that they cannot be sufficiently cleaned to maintain desirable color matching, which makes the dentures more visibly apparent (less concealed). As noted above, the polymer compositions described herein have surprisingly improved color stability (*e.g*. anti-staining ability), which can prolong the usable lifetime of the denture frame and reduce the rate at which the dentures are replaced based on the staining of the denture frame.

With respect to mechanical performance, the oral environment is, additionally, a very demanding application setting. The masticatory force generated during routine mastication of food can be from about 70 Newtons ("N") to 150 N, and up to 500 N to 700 N depending on the type of food and muscular size/density. The force is distributed along the anterior, general (covering the entire arch) and posterior parts of the arch formed by the teeth. At the locations of artificial teeth of the denture, the force is also at least partially transferred to the denture frame. Additionally, horizontal forces on the denture frame are generated during mastication by occlusal contact and by the oral musculature surrounding the denture during mastication. Such forces can displace the denture and denture frame in both antero-posterior and lateral directions as well as place tremendous impact forces on the denture frame. After repeated use, the denture frame can suffer mechanical failure. Relative to polymer compositions including only PEEK polymers, the polymer compositions have improved toughness and flexibility, allowing for denture frames having increased lifetimes due to increased mechanical performance.

Still further, the denture frames described herein have improved comfort, relative to denture frames having a polymer compositions including PEEK polymers alone. In conjunction with the improved mechanical properties described above, the polymer compositions described herein allow for denture frames having thinner components while maintaining desirable mechanical properties. Not only are the resulting denture frames lighter, the thinner components allow for denture frames less noticeable to the wearer with respect to feel. Moreover, as discussed in detail below, it was surprisingly found that the denture frames described herein, when fabricated using selected milling methods, had significantly improved dimensional stability, relative to corresponding denture frames fabricated with injection molding methods. Accordingly, patient fitment issues are reduced.

### The Polymer Composition

The denture frames include a polymer composition containing at least one PEEK polymer and at least one PPSU polymer. In some embodiments, the polymer composition further includes one or more additives. In some embodiments, each of the polymers in the polymer composition (or in the denture frame) is a PEEK polymer or a PPSU polymer.

As mentioned above, the polymer compositions have surprising color retention properties. In particular, relative to corresponding polymer compositions free of the PPSU polymer, the polymer compositions described herein have improved color retention. For clarity, a corresponding polymer composition is one in which the PPSU polymer is replaced with PEEK polymer. For example, if a polymer composition includes at least one PEEK polymer, at least one PPSU polymer and additives, the corresponding polymer composition is the one in which the at least one PPSU polymer is replaced with the at least one PEEK polymer.

The denture frames may consist essentially of the polymer composition, with respect to color stability or dimensional stability.

The ratio of the concentrations of the at least one PEEK polymer to the at least on PPSU polymer can be 40/60 to 90/10, preferably 50/50 to 80/20, preferably 55/45 to 75/25, preferably 58/42 to 70/30, most preferably 63/37.

The polymer composition includes at least one PEEK polymer. As used herein, a PEEK polymer denotes any polymer having, relative to the total number of moles of recurring units, at least 50 mol% of a recurring unit (R_{PEEK}) represented by the following formula: 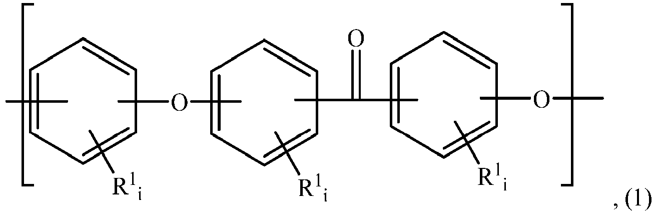 where R¹, at each instance, is independently selected from the group consisting of a halogen, an alkyl, an alkenyl, an alkynyl, an aryl, an ether, a thioether, a carboxylic acid, an ester, an amide, an imide, an alkali or alkaline earth metal sulfonate, an alkyl sulfonate, an alkali or alkaline earth metal phosphonate, an alkyl phosphonate, an amine and an quaternary ammonium; and i, at each instance, is an independently selected integer from 0 to 4. In some embodiments, each i is zero. For clarity, in Formulae (1), each of the benzene rings has 4 - i ring carbons bonded to a hydrogen atom, where i is from 0 to 4, selected independently for each i in Formula (1). For example, referring to Formula (1), if i = 1 for the left most benzyl ring, 3 of those benzyl carbons are bonded to a hydrogen and one is bonded to an R¹. Analogous notation is used for the other formulae herein. In some embodiments, recurring unit (R_{PEEK}) is represented by the following formula:

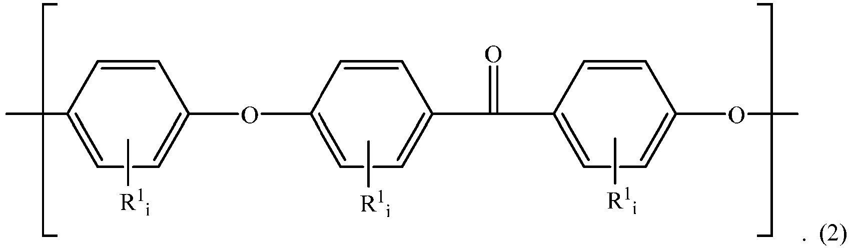

In some such embodiments, each i is zero.

In some embodiments, the PEEK polymer has at least about 60 mol%, at least about 70 mol%, at least about 80 mol%, at least about 90 mol%, at least about 95 mol% or at least about 99 mol% recurring unit (R_{PEEK}), relative to the total number of moles of recurring units in the PEEK polymer. In some embodiments, the at least one PEEK polymer includes one or more recurring units (R^{∗}_{PEEK}), in addition to recurring unit (R_{PEEK}). Each of the one or more recurring units (R^{∗}_{PEEK}) is represented by Formula (1) or (2), and is distinct from each of the other recurring units in the polymer. In such embodiments, the total concentration of the one or more recurring units (R^{∗}_{PEEK}) and the recurring unit (R_{PEEK}) is more than about 50 mol%, at least about 60 mol%, at least about 70 mol%, at least about 80 mol%, at least about 90 mol%, at least about 95 mol% or at least about 99 mol%, relative to the total number of moles of recurring units in the PEEK polymer.

In some embodiments, the concentration of the at least one PEEK polymer, relative to the total weight of the polymer composition, is at least about 30 wt.%, at least about 40 wt.%, at least about 50 wt.% or at least about 55 wt.%. Additionally or alternatively, the concentration of the at least one PEEK polymer, relative to the total weight of the polymer composition, is no more than about 80 wt.%, no more than about 75 wt.% no more than about 70 wt.% or no more than about 65 wt.%. The person of ordinary skill in the art will recognize that additional PEEK polymer concentrations within the explicitly disclosed ranges are contemplated and within the scope of the present disclosure. For clarity, in embodiments in which the at least one PEEK polymer includes a plurality of PEEK polymers, the total concentration of the PEEK polymers in the polymer composition is within the ranges described above.

### The Polyphenylsulfone Polymer

The polymer compositions includes at least one PPSU polymer. As used herein, a PPSU polymer denotes any polymer having, relative to the total number of moles of recurring units, at least 50 mol % of a recurring unit (R_{PPSU}) represented by the following formula: 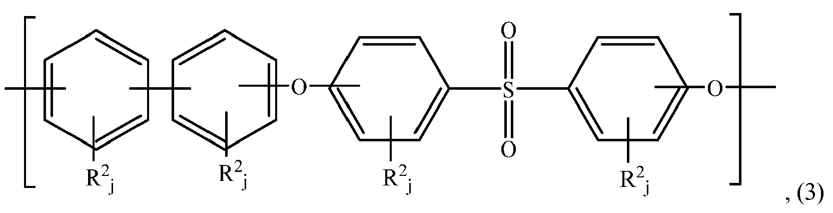 where R², at each instance, is independently selected from a halogen, an alkyl, an alkenyl, an alkynyl, an aryl, an ether, a thioether, a carboxylic acid, an ester, an amide, an imide, an alkali or alkaline earth metal sulfonate, an alkyl sulfonate, an alkali or alkaline earth metal phosphonate, an alkyl phosphonate, an amine, and a quaternary ammonium; and j, at each instance, is an independently selected integer from 0 to 4. Preferably each j is zero. In some embodiments, recurring unit (R_{PPSU}) is represented by the following formula: 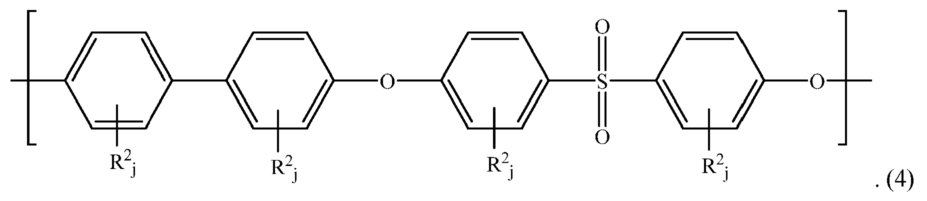 In some such embodiments, each j is zero.

In some embodiments, the PPSU polymer has at least about 60 mol%, at least about 70 mol%, at least about 80 mol%, at least about 90 mol%, at least about 95 mol% or at least about 99 mol% recurring unit (R_{PPSU}), relative to the total number of moles of recurring units in the PPSU polymer. In some embodiments, the at least one PPSU polymer includes one or more recurring units (R^{∗}_{PPSU}), in addition to recurring unit (R_{PPSU}). Each of the one or more recurring units (R^{∗}_{PPSU}) is represented by Formula (1) or (2), and is distinct from each of the other recurring units in the polymer. In such embodiments, the total concentration of the one or more recurring units (R^{∗}_{PPSU}) and the recurring unit (R_{PEEK}) is more than about 50 mol%, at least about 60 mol%, at least about 70 mol%, at least about 80 mol%, at least about 90 mol%, at least about 95 mol% or at least about 99 mol%, relative to the total number of moles of recurring units in the PPSU polymer.

In some embodiments, the concentration of the at least one PPSU polymer, relative to the total weight of the polymer composition, is at least about 10 wt.%, at least about 15 wt.%, at least about 20 wt.%, at least about 25 wt.% or at least about 30 wt.%. Additionally or alternatively, the concentration of the at least one PPSU polymer, relative to the total weight of the polymer composition, is no more than about 60 wt.%, no more than about 50 wt.%, no more than about 55 wt.%, no more than about 50 wt.%, no more than about 45 wt.% or no more than about 40 wt.%. The person of ordinary skill in the art will recognize that additional PPSU polymer concentration ranges within the explicitly disclosed ranges are contemplated and within the scope of the present disclosure. For clarity, in embodiments in which the at least one PEEK polymer includes a plurality of PEEK polymers, the total concentration of the PEEK polymers in the polymer composition is within the ranges described above.

### Additives

As noted above, in some embodiments, the polymer composition can include one or more additives. The additives can be selected from the group consisting of ultraviolet light stabilizers, antioxidants, pigments, processing aids, lubricants, and radiopaque compounds (including, but not limited to, barium sulfate, bismuth trioxide, bismuth oxychloride, and bismuth subcarbonate.

Pigments can be particularly desirable additives in the polymer composition, to impart desirable aesthetic qualities to the denture frame. In light of the desire for surreptitious use, aesthetics are a significant consideration with respect to denture frames. The more the denture frame can be hidden from casual sight and the more the frame blends into the oral environment, the more concealed the use of the denture frame (and the ultimate denture). Pigments incorporated into the polymer composition of the denture frame to impart aesthetic qualities to help conceal use in the oral environment can include, but are not limited to, TiO₂ (*e.g.* rutile, anatase or brookite) (white), coumarin (yellow), lapis lazul (blue) or any combination of two or more thereof. In embodiments in which the polymer composition includes a pigment, the total concentration of pigments, relative to the total weight of the polymer composition, can be at least about 0.1 parts per hundred by weight ("pph"), at least about 1 pph, at least about 1 pph, at least about 2 pph or at least about 3 pph. In some embodiments, the total concentration of pigments, relative to the total weight of the polymer composition, is no more than about 25 pph, no more than about 15 pph, no more than about 10 pph or no more than about 7 pph. A person of ordinary skill in the art will recognize that additional ranges of total pigment concentration within the explicitly disclosed ranges is contemplated and within the scope of the present disclosure.

Significantly, applicants discovered that inclusion of particulate additives at relatively high loading levels can cause premature breakage of the frame. One class of particulate additives is inorganic particles having an average primary particle diameter of 100 including, but not limited to, TiO₂. Particulate additives have a general spherical appearance. Upon close examination, crystalline particulate additives, such as inorganic particles, have facets corresponding to the underlying crystal lattice, but nevertheless have roughly equivalent spatial dimensions from the geometric center. As described below, portions of the denture frames of interest herein are relatively thin (e.g. having a width less than 5 mm or even less than 2 mm). Frequent insertion and removal of the denture frame from the oral cavity, as well as mastication, during normal use places a significant amount of flexural strain on the denture frame. Applicant found that inclusion of particulate additives at relative high loading levels compromises the mechanical integrity of the denture frame and significantly reduces the lifetime of the denture frames. For the denture frames of interest herein, the total concentration of particulate fillers is less than about 30 wt. %, less than about 20 wt. %, less than about 10 wt. %, less than about 5 wt. %, less than about 2 wt. %, relative to the total weight of the polymer composition. The person of ordinary skill in the art will recognize additional particular filler concentrations within the explicitly disclosed ranges are contemplated and within the scope of the present disclosure.

In some embodiments, the particulate additives have an average primary particle diameter (length in the longest dimension of the particle) of from about 100 nanometers ("nm") to about 5 micrometers ("µm"). In such embodiments, the particulate additives can have a distribution of particle diameters such that at least about 80%, at least about 95%, or at least 99% of the primary particles have a diameter greater than about 40% of the average diameter and less than about 700% of the average diameter. In further embodiments, the particulate additives can have a distribution in primary particle diameters such that at least about 80%, at least about 95%, or at least 99%, of the primary particles have a diameter greater than about 40% of the average diameter and less than about 300% of the average diameter. In alternative or additional embodiments, the particulate additives can have a distribution of primary particle diameters such that at least about 95% or at least 99% of the primary particles have a diameter greater than about 45% of the average diameter and less than about 200% of the average diameter. A person of ordinary skill in the art will recognize additional ranges of average primary particle diameter and primary particle diameter distributions within the explicitly disclosed ranges above are contemplated and within the scope of the present disclosure. Primary particle sizes (as well as average primary particle sizes and corresponding distributions) can be determined by transmission electron micrographs ("TEM"). For clarity, "primary" particle refers to the unagglomerated particle. Due to their small size, the primary particles tend to form agglomerates because of vad der Waals forces. Nevertheless, the primary particles can be clearly seen in TEM images.

In some embodiments, the polymer composition is free of a fibrous fillers. Fibrous fillers include, but are not limited to, glass fibers and carbon fibers. The presence of fibrous fillers in oral application settings can present health issues. Accordingly, in some embodiments, the polymer composition has less than about 10 wt.%, preferably less than 5 wt.% of a fibrous filler, relative to the total weight of the polymer composition.

### Fabrication of Denture Frames

The denture frames described herein are desirably fabricated using a milling approach. Desirable milling approaches involve cutting a blank including the polymer composition to produce the denture frame (also known as subtractive manufacturing or machine milling). Desirably, the blanks are formed by extruding the polymer composition into a basic shape *(e.g.* a rod) subsequently cutting the shape to have the desired thickness. Advantageously, the fabrication method is free of injection molding approaches with respect to blank or denture frame fabrication.

As mentioned above it was surprisingly found that denture frames including the polymer composition described herein, when fabricated using selected milling methods, had significantly improved dimensional stability, relative to corresponding denture frames fabricated with injection molding methods. In injection molding, the molten polymer composition is injected into a mold having an inner cavity forming the negative of the intended denture frame design or injection molded into a mold having an inner cavity forming a blank and subsequently milled into a denture frame (described in detail below). It was surprisingly found that the polymer composition of the denture frame fabricated with injection molding techniques exhibited significantly compromised dimensional stability and, correspondingly, the dimensional fidelity of the denture frame with its original, intended design. In at least some instances, the loss of fidelity resulted in eventual inadequate fitment of the denture frame to the extent that it was an unacceptable for use in the patient's mouth. On the other hand, denture frames fabricated using a milling approach had significantly increased dimensional stability and corresponding fidelity to the design of the denture frame.

A desirable milling approach involves cutting an extruded blank (as described below) formed from the polymer composition to produce the denture frame. During milling, a cutting tool is used to remove material of the blank to form the denture frame. In one embodiment, the cutting tool has cutting edges *(e.g.* a drill bit including, but not limited to, a router bit) that are contacted with the blank to remove material of the blank corresponding to the negative design of the denture frame. Based on the disclosure herein, the person of ordinary skill in the art will know how to select an appropriate cutting tool as well as use parameters such as rotation frequency and routing speed according the specific denture frame features and polymer composition. In other embodiments, a laser can be used as a cutting tool. Based on the disclosure herein, the person of ordinary skill in the art will know to appropriately select a laser and use parameters such as pulse rate and raster speed according to the specific denture frame features and polymer composition.

In some embodiments, the cutting tool can be desirably controlled using a computer processor. In such embodiments, the computer processor can be in electronic communication with one or more controllers that move the cutting tool and control its use parameters (*e.g*. rotation speed of a drill bit). The computer processor can also be in electronic communication with a memory (*e.g*. processor cache, random access memory or other physical memory including, but not limited to, a hard drive, a solid state drive, and universal serial bus storage) containing a digital representation of the denture frame. The computer processor can access the memory and control the positioning, as well as the use parameters of the cutting tool, to remove polymer composition from the blank and form the desired denture frame. Examples of such computer aided milling approaches include, but are not limited to, CAD/CAM, in which a computer aided design ("CAD") software is used to create digital file containing a digital representation of the denture frame, readable by a computer processer, and a computer aided manufacturing ("CAM") is used to read the digital file and control the cutting tool to fabricate the denture frame as described above according to the digital representation. Machines for implementing CAM methods moving the cutting tool or object to be milled in various directions. CAM machines can be 3-axis (corresponding to the 3 translation axes), 4-axis to 6-axis (3 translation axes + 1 to 3 rotational axis) or 7-axis apparatuses. Five-axis and 7-axis CAM machines can be particularly desirable in light of the complicated design features of a denture frame. In some embodiments, the digital representation can be obtained using a digital file containing a digital representation of a patient's mouth, for example, obtain by a direct optical scan of the patient's mouth or an optical scan of a mold of a patient's mouth. Using the scan, the denture frame design (*e.g*. physical dimensions and features of the denture frame), and corresponding digital file, can be produced by using CAD.

The blank is a solid block of the polymer composition. The blank can be any shape or size suitable for use with a milling machine. In some embodiments, cylindrical blanks (also known as pucks) can be desirably used. In some such embodiments, the cylindrical blank has a thickness ranging from about 10 millimeters ("mm") to about 70 mm or from about 15 mm to 60 mm, and a diameter ranging from about 20 mm, from about 40 mm or from about 70 mm to about 100 mm. The person of ordinary skill in the art will recognize additional ranges of thickness and diameter within the explicitly disclosed ranges are contemplated and within the scope of the present disclosure. The blank can be made by extruding the polymer composition. In some such embodiments, the polymer composition is extruded into rods having the desired diameter of the cylindrical blank and the rod is subsequently cut perpendicular to the direction of extrusion to form pucks have the desired thickness ("extruded blank"). In some embodiments, the polymer composition can be cut as it exits the extruder. In other embodiments, rods can be formed having a length larger than the blank and subsequently cut to form the cylindrical blanks. As noted above, while blanks may also be formed by injection molding the polymer composition into a mold having an inner cavity corresponding to the desired blank dimensions, denture frames milled from such blanks have significant dimensional instability.

### Denture Frames and Dentures

The denture frame may be incorporated into a denture. The denture may be a complete denture that replaces all of a patient's teeth in a single arch (e.g. the maxillary (upper) or mandibular (lower) arch), or a partial denture, which replaces less than all of a patient's teeth in a single arch. Thus, when the denture is a partial denture, it is designed to accommodate a patient's existing teeth or implants. In some aspects, the denture is a partial removable denture that is designed to be regularly removed from the patient's mouth for cleaning.

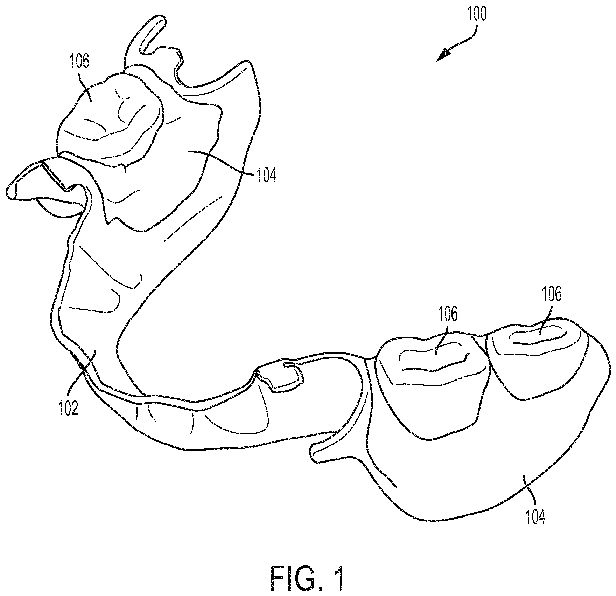

Referring to Fig. 1, the components of the denture 100 include a denture frame 102, artificial gums 104 supported by the denture frame 102, and artificial teeth 106 supported by the artificial gums 104. The artificial gums 104 preferably include at least one acrylic polymer, and may be colored to match a patient's gums and mimic natural gums. Similarly, the artificial teeth 106 mimic the shape and color of natural teeth.

In some aspects, the dental prosthesis is a denture frame, preferably a partial removable denture frame. The denture frame may be formed from a single piece of plastic and may be free of metal. As used herein, a denture frame that is free of metal includes less than 1 % of metal by weight of the denture frame. As used herein, "metal" means elemental metals or alloys thereof such as, for example, gold, silver, platinum, nickel, aluminium, stainless steel, etc.

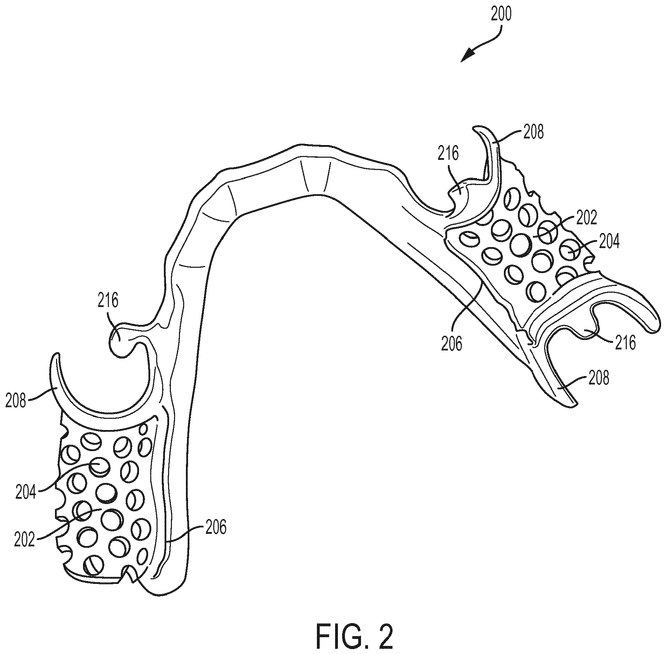

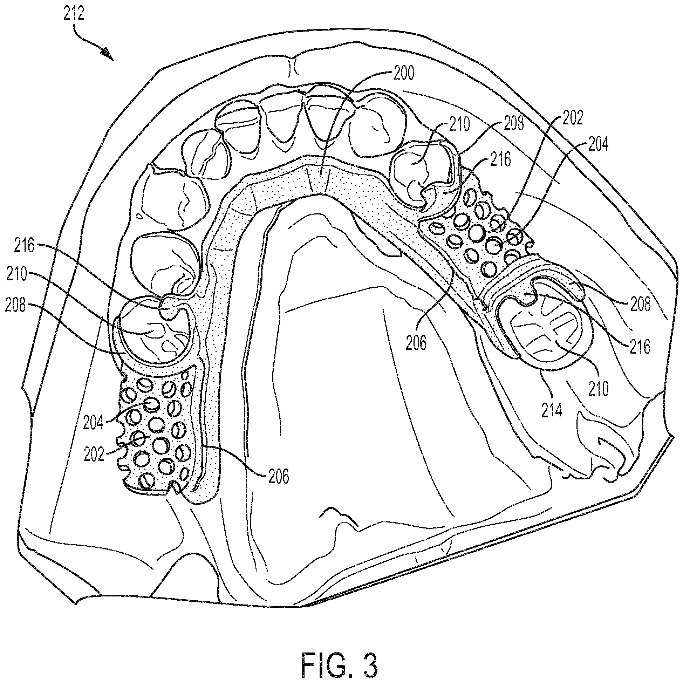

In some cases, at least a portion of the polymer composition in the denture frame has a crystallinity greater than 21 %, and the polymer composition includes less than 63 wt. % of the PEEK based on the total weight of the polymeric material, where the crystallinity is determined by measurement of the enthalpy of fusion from the second heat cycle by differential scanning calorimetry (DSC) according to ASTM D3418-03, E1356-03, E793-06, and E794-06. Referring to Figs. 2 and 3, denture frame 200 includes retention grid 202, which is a portion of the denture frame adapted for attachment of the artificial gums (not shown). The retention grid includes one or more retention holes 204 extending through denture frame 200 to aid in the attachment of the artificial gums. The holes can have an area (*e.g*. opening diameter) of less than 10 square millimeters ("mm"), less than 7 mm², or less than 5 mm². The denture frame may include at least two, preferably at least four, preferably at least six, preferably at least 8 or more holes. The holes may be positioned within the retention grid to key artificial gum material onto the denture frame. In other words, when the artificial gums are attached to the denture frame, the material of the artificial gums may extend through the holes in the retention grid to aid in mechanical adhesion of the artificial gums to the denture frame. Depending on the needs of the patient, the retention grid can be flat or curved to adhere to the shape of the patient's existing natural gum tissue.

The denture frame includes a finish line. Referring again to Figs. 2 and 3, finish line 206 is a ridge in denture frame 200 that bounds the retention grid 202 on one or more sides of retention grid 202. When the artificial gums (not shown) are attached to denture frame 200, finish line 206 extends along and interfaces with the a border of the artificial gums where the artificial gums contact denture frame 200.

Applicant found that, in conjunction with the polymer composition having improved flexibility and durability, denture frames having finish lines with a substantially flat inner surface have significantly improved structural integrity when incorporated into a denture including an artificial gum. As described above, a denture includes a denture frame having a finish line in contact with an artificial gum. The finish line has a tip, disposed towards the top of the denture frame, and a base, disposed towards the bottom of the denture frame. The two surfaces between the top and base of the finish line are referred to as the inner surface (the surface configured to contact the artificial gum when the denture frame is assembled into a denture) and the outer surface. The finish lines of interest herein have a substantially flat inner surface, which flexes with the artificial gum when in use in the mouth. Relative to finish line designs in which the inner surface is cupped to help to retain the artificial gum in the denture frame, the stress placed on the finish lines of interest herein resulting from flexing is significantly reduced, due to the substantially flat inner surface, as described in more detail below. As used herein, a substantially flat inner surface refers to a surface that is oriented within 20 degrees of an axis that is perpendicular to (i) to the base and (ii) the direction of the finish line; ("Reference Axis"). The inner surface is oriented within 20 degrees of the Reference Axis over at least 85 % over the surface including the tip and extending towards the base. In some embodiments, the inner surface is oriented within 20 degrees of the Reference Axis over at least 90 %, at least 95 %, or at least 99 % over the surface including the tip and extending towards the base. In some embodiments, the finish line has a linear distance from the tip to the base in a cross section of the finish line from about 0.5 mm to about 1.5 mm, over at least 90 %, at least 95 % or at least 99 % of the length of the finish line. In some embodiments, the finish line has a substantially flat inner surface along at least 90 %, at least 95 %, or at least 99 % of the length of the finish line. The length of the finish line is its length along the tip from endpoint to endpoint (e.g. start to finish).

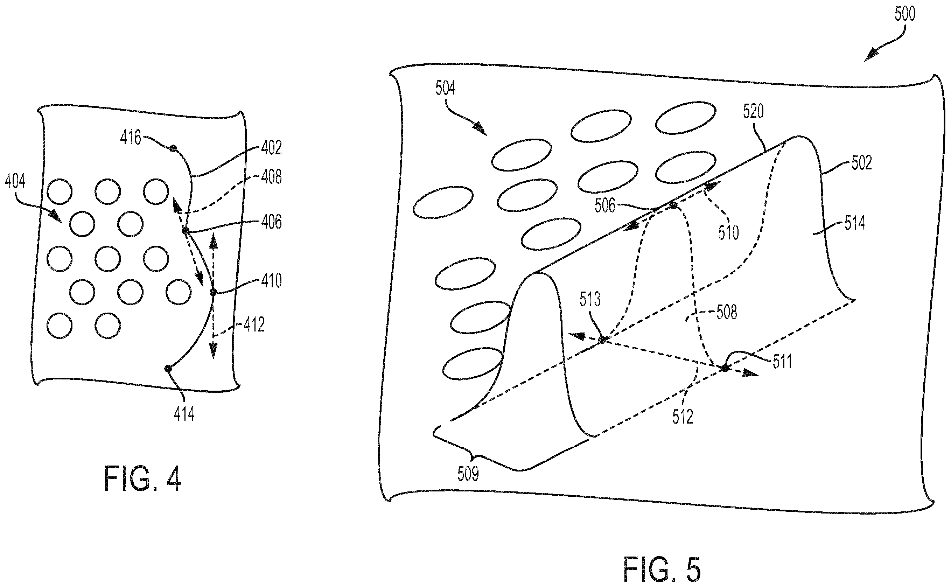

The substantially flat surface can be further understood by looking at a cross section of the finish line perpendicular to its direction. The direction of the finish line can be determined by viewing a denture frame in a top down orientation. In such a perspective, the tip of the finish line traces out a curve. At any point along the finish line (e.g. the point at which a cross section is taken), its direction is oriented along the tangent line at that point. Fig. 4 is a schematic depiction showing a top-down view of a portion of a denture frame showing a finish line. Referring to Fig. 4, finish line 402 is adjacent to retention grid 404. The orientation of finish line 402 at point 406 is along direction 408. Similarly, the orientation of finish line 402 at point 410 is along direction 412. The length of finish line 402 is its length from endpoint 414 to endpoint 416.

With respect to the cross section of the finish line, Fig. 5 is a schematic depiction of a perspective view of a region of a denture frame showing a finish line. Denture frame region 500 has finish line 502 adjacent retention grid 504. At point 506, finish line 502 has cross section 508, which is perpendicular to the direction of finish line axis 510. Axis 512 is in the plane of cross section 508, and intersects base 509 of finish line 502 at point 511 on outer surface 514 and at point 513 on the inner surface (not labelled).

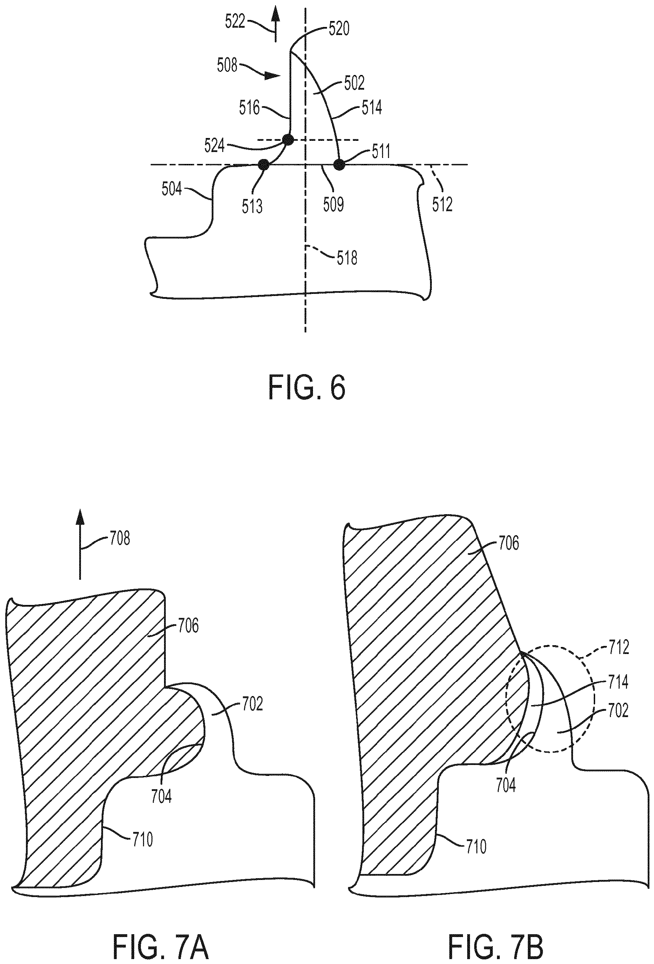

Referring to Fig. 6, finish line 502 has outer surface 514 and inner surface 516. Outer surface 514 is oriented away from retention grid 504 and inner surface 516 is oriented towards retention grid 504. Inner surface 516 is a substantially flat surface. Inner surface 516 is oriented within 20 degrees of Reference Axis 518 over at least 85 %, at least 90 %, at least 95 %, or at least 99 % over the region of inner surface 516 extending from tip 520 and towards base 509. In some embodiments, inner surface 516 is substantially flat along at least 90 %, at least 95 %, or at least 99 % of the length of finish line 502.

The substantially flat inner surface allows greater flexing of the finish line without breaking, relative to alternative finish line designs. In particular, alternative finish lines having a cupped inner surface are widely used in thermoplastic denture frames, at least because (a) they help to hold the artificial gum in place and (b) they help to prevent food and other debris in the mouth from being trapped between the artificial gum and the finish line. Fig. 7 is a schematic depiction of a cross section of a portion of a denture displaying a finish line with a cupped inner surface and a portion of an artificial gum. Referring to Fig. 7A, Finish line 702 has cupped inner surface 704, which helps to retain artificial gum 706. Cupped inner surface 704 helps to restrict movement of gum 706 in direction 708 (opposite to retention grid 710), as well as to help prevent debris (e.g. food) from becoming trapped between artificial gum 706 and finish line 702. However, when used in the mouth, artificial gum 706 and finish line 702 can flex (other components of the denture frame can also flex). It was found that such flexing can place significant stress on finish line 702, which may cause breakage. Fig. 7B depicts finish line 702 upon flexing, as previously described. Because of cupped inner surface 704, as artificial gum 706 flexes, artificial gum 706 and finish line 702 are pushed apart, creating cavity 714 and causing finish line 702 to deform significantly under the applied stress, as schematically depicted by region 712. The applied stress can cause finish line 702 to break, for example in region 712, due to its relatively thin design.

Finish lines incorporating a substantially flat inner surface can significantly reduce the risk of retention line and denture frame breakage. The substantially flat inner surface reduces the stress on the finish line upon flexing of the artificial gum, relative to a design having a cupped inner surface. Because the inner surface does not help to hold the artificial gum in place (e.g. at least partially due to the fact that surface is substantially flat), flexing of the finish line with flexing of the artificial gum is significantly reduced, reducing the stress placed on the finish line. In other words, in the absence of bonding to the retention grid, the artificial gum can be slidably released from the denture frame in a direction opposite from the retention grid (e.g. direction 522 in Fig. 6, parallel to Reference Axis 518), which helps to prevent the finish line from holding the artificial gum in place during flexing. Furthermore, in combination with the polymer composition having improved flexibility, structural integrity under flexing is still further improved.

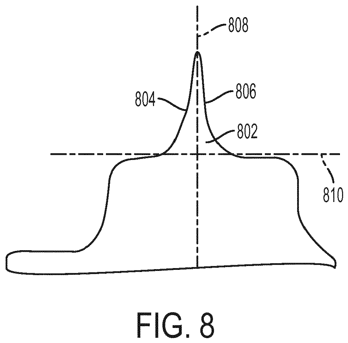

The outer surface of the finish line is generally shaped to form a smooth transition from the base to the artificial gum. In some embodiments, the magnitude (absolute value of) the angle of the outer surface relative to the Reference Axis is less than that of the inner surface, over at least 80 %, at least 90 % or at least 95 % of the region of the inner surface extending from the tip and towards the base. In such embodiments, the finish line has an asymmetric cross section in a plane perpendicular to the length of the finish line (e.g. the cross section lacks an axis of symmetry parallel to the Reference Axis). For example, referring again to Figs. 5 and 6, outer surface 514 provides a smooth transition from base 509 (at point 511) to tip 520. The magnitude of the angle between outer surface 514 and Reference Axis 518 is greater than the magnitude of the angle between inner surface 516 and Reference Axis 518 over the region of inner surface 516 from tip 520 to location 524 (at least 80 %, at least 90 % or at least 95 % of the region of the inner surface extending from the tip and towards the base). Moreover, cross section 508 of finish line 502 depicted lacks an axis of symmetry parallel to Reference Axis 518. In other embodiments, the magnitude of the angle of the outer surface relative to the Reference Axis is substantially the same (within 10°) as that of the inner surface from the tip to the base. In such embodiments, the finish line has a symmetric cross section in a plane perpendicular to the length of the finish line (e.g. the cross section has an axis of symmetry parallel to the Reference Axis). For example, Fig. 8 is schematic depiction of a cross section of a portion of a denture frame showing a finish line having a symmetric cross section. Finish line 802 has substantially flat inner surface 804 and outer surface 806. The magnitude of the angle of outer surface 806 with Reference Axis 808 is substantially the same magnitude of the angle of inner surface 804 with Reference Axis 808. Moreover, cross section of finish line 802 has an axis of symmetry along Reference Axis 808. Reference Axis 808 is perpendicular to axis 810.

The denture frame may also include rests or clasps, which in the context of a partial removable denture frame, anchor the denture frame in the patient's mouth by friction fitting the denture frame to the patient's existing natural teeth or implants. Applicants have surprising found that denture frames made from the polymer composition exhibit increased toughness, flexibility, and dimensional stability allowing for the use of clasps and rests that improve fit and retention of the denture frame.

Referring again to Figs. 2 and 3, In some embodiments, denture frame 200 may include at least one clasp 208 that that extends from denture frame 200 to wrap around and grip undercut 214 of patient's natural tooth 210 or implant (not shown), thereby friction fitting denture frame 200 in patient's mouth 212.

It was discovered that the increased flexibility and reduced brittleness of the polymer composition allows design of clasps that are more durable and fit farther into the undercut of a patient's existing teeth or implants to provide better fit and retention than has previously been possible with, for example, metal denture frames or denture frames having a corresponding polymer compositions containing PEEK as the only polymeric component of the polymer composition.

Referring again to Figs. 2 and 3, in some embodiments, denture frame 200 includes rests 216 that aid in holding denture frame 200 in position in patient's mouth 212. A rest is a portion of the denture frame extending onto the bite surface of a patient's natural tooth or implant. Because the rest may come into contact with an opposing tooth during chewing, it must be impact resistant and can become abraded. It was found that the polymer composition is particularly well-suited for use in rests because of its toughness and flexibility.

### EXAMPLES

The invention will be now described in more detail with reference to the following examples, whose purpose is merely illustrative.

### Starting Materials

The following materials were used to prepare the Examples :
KetaSpire^{®} PEEK KT-820 NL Q available from Solvay Specialty Polymers USA, L.L.C.
Radel^{®} PPSU R-5000 NT and R-5100 P NT available from Solvay Specialty Polymers USA, L.L.C.
Titanium dioxide (TiO2) - Grade : TiPure^{®} R105 available from Chemours.

### Blend Preparation

Each formulation was melt compounded using a 26 mm diameter Coperion^{®} ZSK-26 corotating partially intermeshing twin screw extruder having an L/D ratio of 48:1.

In each case, the resins and additives were fed at barrel section 1 using a gravimetric feeder at throughput rates in the range 30-40 lb/hr. The extruder was operated at screw speeds of around 200 RPM. Vacuum was applied at barrel zone 10 with a vacuum level of about 27 inches of mercury. A single-hole die was used for all the compounds and the molten polymer strand exiting the die was cooled in a water trough and then cut in a pelletizer to form pellets approximately 3.0 mm in length by 2.7 mm in diameter.

### Injection Molding

The example formulations were injection molded to produce 3.2 mm (0.125 in) thick ASTM tensile and flexural specimens for mechanical property testing. Type I tensile ASTM specimens and 5 in x 0.5 in x 0.125 in flexural specimens were injection molded.

### Mechanical Testing

Mechanical properties were tested using injection molded test specimens which consisted of 1) ISO bars 80 x 10 x 4 mm, and 2) 2 x 3 x 0.125 in plaques. The following test methods were employed in evaluating the compositions :
ASTM D-638 : Tensile properties : tensile strength at yield, tensile modulus and tensile elongation at yield
ASTM D790 : flexural properties
ASTM D792 : density and specific gravity
ASTM D256 : Notched Izod impact resistance

The results of the mechanical testing are shown below in Table 1.

**Table 1**

| **Example No.** | **C1** | **E1** | **E2** | **E3** |
|---|---|---|---|---|
| PEEK, KetaSpire^{®} KT-820 NL Q | 100 | 62.9 | 62.9 | 62.9 |
| PPSU, Radel^{®} R-5100 P NT | -- | 36.9 | 36.9 | 6.9 |
| PPSU, Radel^{®} R-5000 NT | -- | -- | -- | 30 |
| Zinc oxide | -- | 0.1 | 0.1 | 0.1 |
| Zinc stearate | -- | 0.1 | 0.1 | 0.1 |
| TiO₂, TiPure^{®} R-105 (pph) | -- | 0 | 3 | 5 |
| Color concentrate package (pph) | -- | -- | 0.024 | 0.018 |
| | | | | |
| Density (g/cm) | -- | 1.28-1.32 | 1.32 | -- |
| Tensile Strength at yield (MPa) | -- | 84 | 87 | 87.8 |
| Tensile Modulus (GPa) | 3.5 | 3.1 | 3.22 | 3.18 |
| Tensile Elongation at Yield (%) | 5.2 | 4 | 6.1 | 6.19 |
| Tensile Elongation at Break (%) | 78 | 30 | -- | 90 |
| Flexural Modulus (GPa) | 3.7 | 3.1 | -- | 3.25 |
| Flexural Strength (MPa) | 146 | 122 | -- | 128.9 |
| Notched Izod Impact (J/m) | 91 | 100 | 100 | 98.2 |

### Dimensional Stability Testing

The dimensional stability of denture frames made from extruded and injection molded cylindrical blanks of the composition of Example 3 was assessed.

The composition of Example 3 was injection molded into cylindrical blanks (i.e. blanks) measuring 98 mm in diameter and 18 mm in thickness. Blanks of identical size were also prepared by extruding of a rod of the composition of Example 3 and cutting the rod to form extruded blanks.

A mandibular impression was taken of a patient's teeth, from which a plaster cast was prepared. The plaster cast was scanned using a 3Shape D750 Lab Scanner to create an electronic model of the patient's teeth. A denture frame was designed using a computer employing CAD/CAM technology and identically-shaped denture frames were milled from each blank. Following milling, the fit of each denture frame was assessed on the cast model by visual inspection. A framework was considered seated when all rests on the denture frame came into full contact with their rest seats on the cast model. Each denture frame fit well on the cast model directly after milling. After approximately 24 hours, the fit of each denture frame was reassessed by visual inspection. While the denture frames milled from extruded blanks continued to exhibit good fit, the denture frames milled from injection molded blanks were found to have distortions of over 2 mm from their original dimensions, rending the frames unusable.

Accordingly, it was unexpectedly discovered that dimensional stability is increased and fit is maintained when the denture frames are milled from extruded blanks as compared with injection molded blanks.

### Color Stability Testing

The color stability of the compositions of Example 3 and Comparative Example C1 after exposure to coffee (a typical staining agent found in the oral environment) was evaluated using a modified AL-PCL-MEC-LTM-077 test method.

Six test specimens in the form of color chips were prepared from each of the materials by injection molding.

A coffee staining solution was prepared by adding 20 g Nescafe ClasicoTM dark roast coffee, available from Nestle, to 1000 ml of boiling distilled water.

Color change for each specimen was evaluated using an XRite^{®} Color i7800 spectrophotometer. The spectrophotometric reflectance was measured from 360 - 750 nm, with measurements on each test specimen made in triplicate.

Each specimen was conditioned by placing it in distilled water at 37+/-1°C for 24 hours before spectrophotometric data was collected as a baseline measurement. Following conditioning, three test specimens of each material were soaked in the coffee staining solution, and the remaining three specimens were soaked in distilled water as a control, at 37+/-1°C for 30 days. The test specimens were removed after 30 days and analyzed with the spectrophotometer. Each test specimen was cleaned by placing it in a Ney Ultrasonic 28B cleaner for 10 minutes at room temperature (21°C). The cleaning solution was Ultrasonic Solution #4 Tartar and Stain Remover available from Quala Dental Products. Spectral analysis was also performed following cleaning.

The color was measured according to the CIE 1976 L-a-b coordinates standard where the L^{∗} coordinate represents the lightness (black to white) scale, the a^{∗} coordinate represents the green-red chromaticity and the b^{∗} scale represents the blue-yellow chromaticity. Delta E [ΔE=((ΔL)2 + (Δa)2 + (Δb)2)1/2] values were calculated from the spectrophotometer results as the difference between each reading and the baseline measured after conditioning and prior to staining. The ΔE value was used to assess the color stability, with higher values indicating a higher level of staining.

The results of the color stability testing are shown below in Table 2.

**Table 2**

| | | T1 30 days ΔE | T2 After Cleaning ΔE | Difference After Cleaning (T1-T2) |
|---|---|---|---|---|
| Composition of Comparative Example C 1 | | | | |
| **C2** | Coffee | 5.861 | 4.100 | 1.761 |
| **C4** | Water | 0.676 | 0.552 | 0.123 |
| Composition of Example E3 | | | | |
| **E4** | Coffee | 17.557 | 0.410 | 17.146 |
| **C5** | Water | 0.544 | 0.366 | 0.178 |

As shown in Table 2, the compositions of Example 3 and Comparative Example 1 each exhibited increased staining after 30 days in the coffee staining solution as shown by the ΔE_{S} of 17.557 and 5.861, respectively. After cleaning, however, the composition of Example 3 unexpectedly exhibited a significantly greater reduction in staining and a lower ΔE than the composition of Comparative Example 1.

Should the disclosure of any patents, patent applications, and publications which are incorporated herein by reference conflict with the description of the present application to the extent that it may render a term unclear, the present description shall take precedence.

## Claims

1. A denture frame comprising:
a polymer composition comprising:
(i) from about 30 wt.% to about 80 wt.% of at least one poly(ether ether) ketone ("PEEK") polymer, relative to the total weight of the polymer composition;
(ii) from about 10 wt.% to about 60 wt.% of at least one polyphenylsulfone ("PPSU") polymer, relative to the total weight of the polymer composition; and
(iii) less than about 30 wt.% of a particulate filler comprising a pigment,
wherein the denture frame comprises at least one finish line including a substantially flat inner surface.

2. The denture frame of claim 1, wherein the particulate filler as an average primary particle diameter of 100 nm to 5 µm.

3. The denture frame of any one of claims 1 and 2, wherein the particulate filler comprises TiO₂.

4. The denture frame of any one of claims 1 to 3, wherein the polymer composition is free of a fibrous filler.

5. The denture frame of claim 1, wherein the finish line has an asymmetric cross section in a plane perpendicular to the length of the finish line.

6. The denture frame of any one of claim 6, further comprising a retention grid comprising at least one retention hole having an area of less than about 10 mm².

7. The denture frame of any one of claim 1 to 6, further comprising a rest.

8. The denture frame of any one of claims 1 to 7, further comprising a clasp.

9. A denture comprising the denture frame of any one of claims 6 to 8, wherein the denture comprises an artificial gum disposed on the retention grid and in contact with the inner surface of the finish line.

10. The denture of claim 9, further comprising at least one artificial tooth in contact with the artificial gum.

11. A method of forming a denture frame according to anyone of claims 1 to 10, the method comprising:
milling a denture frame from a blank comprising a polymer composition, wherein the polymer compositions comprises:
(i) from about 30 wt.% to about 80 wt.% of at least one poly(ether ether) ketone ("PEEK") polymer, relative to the total weight of the polymer composition;
(ii) from about 10 wt.% to about 60 wt.% of at least one polyphenylsulfone ("PPSU") polymer, relative to the total weight of the polymer composition; and
(iii) less than about 30 wt.% of a particulate filler comprising pigment,
and wherein the denture frame comprises at least one finish line including a substantially flat inner surface.

12. The method of claim 11, wherein the blank comprises a cylindrical blank.

13. The method of claim 12, wherein the cylindrical blank has a thickness from about 10 mm to about 70 mm and a diameter about 20 mm to about 100 mm.

14. The method of any one of claims 12 and 13, further comprising fabricating the blank, wherein the fabricating comprises extruding the polymer composition into a rod having a diameter from about 20 mm to about 100 mm and cutting the rod to form the cylindrical blank.

## Patentansprüche

1. Zahnprothesengerüst, umfassend:
eine Polymerzusammensetzung, umfassend:
(i) von etwa 30 Gew.-% bis etwa 80 Gew.-% zumindest eines Poly(etherether)keton-("PEEK")-Polymers, bezogen auf das Gesamtgewicht der Polymerzusammensetzung;
(ii) von etwa 10 Gew.-% bis etwa 60 Gew.-% zumindest eines Polyphenylsulfon-("PPSU")-Polymers, bezogen auf das Gesamtgewicht der Polymerzusammensetzung; und
(iii) weniger als etwa 30 Gew.-% eines partikulären Füllstoffs, der ein Pigment umfasst,
wobei das Zahnprothesengerüst zumindest eine Präparationsgrenze umfasst, die eine im Wesentlichen flache Innenfläche beinhaltet.

2. Zahnprothesengerüst nach Anspruch 1, wobei der partikuläre Füllstoff einen durchschnittlichen Primärpartikeldurchmesser von 100 nm bis 5 µm aufweist.

3. Zahnprothesengerüst nach einem der Ansprüche 1 und 2, wobei der partikuläre Füllstoff TiO₂ umfasst.

4. Zahnprothesengerüst nach einem der Ansprüche 1 bis 3, wobei die Polymerzusammensetzung frei von einem Faserfüllstoff ist.

5. Zahnprothesengerüst nach Anspruch 1, wobei die Präparationsgrenze einen asymmetrischen Querschnitt in einer Ebene senkrecht zur Länge der Präparationsgrenze aufweist.

6. Zahnprothesengerüst nach Anspruch 6, ferner umfassend ein Retentionsgitter, das zumindest ein Retentionsloch mit einer Fläche von weniger als etwa 10 mm² umfasst.

7. Zahnprothesengerüst nach einem der Ansprüche 1 bis 6, ferner umfassend eine Auflage.

8. Zahnprothesengerüst nach einem der Ansprüche 1 bis 7, ferner umfassend eine Klammer.

9. Zahnprothese, umfassend das Zahnprothesengerüst nach einem der Ansprüche 6 bis 8, wobei die Zahnprothese ein künstliches Zahnfleisch umfasst, das auf dem Retentionsgitter angeordnet ist und mit der Innenoberfläche der Präparationsgrenze in Kontakt ist.

10. Zahnprothese nach Anspruch 9, ferner umfassend zumindest einen künstlichen Zahn, der mit dem künstlichen Zahnfleisch in Kontakt ist.

11. Verfahren zum Bilden eines Prothesengerüsts nach einem der Ansprüche 1 bis 10, das Verfahren umfassend:
Fräsen eines Zahnprothesengerüsts aus einem Rohling, der eine Polymerzusammensetzung umfasst, wobei die Polymerzusammensetzungen Folgendes umfassen:
(i) von etwa 30 Gew.-% bis etwa 80 Gew.-% zumindest eines Poly(etherether)keton-("PEEK")-Polymers, bezogen auf das Gesamtgewicht der Polymerzusammensetzung;
(ii) von etwa 10 Gew.-% bis etwa 60 Gew.-% zumindest eines Polyphenylsulfon-("PPSU")-Polymers, bezogen auf das Gesamtgewicht der Polymerzusammensetzung; und
(iii) weniger als etwa 30 Gew.-% eines partikulären Füllstoffs, der ein Pigment umfasst,
und wobei das Zahnprothesengerüst zumindest eine Präparationsgrenze umfasst, die eine im Wesentlichen flache Innenoberfläche aufweist.

12. Verfahren nach Anspruch 11, wobei der Rohling einen zylindrischen Rohling umfasst.

13. Verfahren nach Anspruch 12, wobei der zylindrische Rohling eine Dicke von etwa 10 mm bis etwa 70 mm und einen Durchmesser von etwa 20 mm bis etwa 100 mm aufweist.

14. Verfahren nach einem der Ansprüche 12 und 13, ferner umfassend das Herstellen des Rohlings, wobei das Herstellen das Strangpressen der Polymerzusammensetzung zu einem Stab mit einem Durchmesser von etwa 20 mm bis etwa 100 mm und das Schneiden des Stabs zur Bildung des zylindrischen Rohlings umfasst.

## Revendications

1. Monture pour prothèse dentaire comprenant :
une composition polymère comprenant :
(i) d'environ 30 % en poids à environ 80 % en poids d'au moins un polymère de poly(éther éther)cétone (« PEEK »), par rapport au poids total de la composition polymère ;
(ii) d'environ 10 % en poids à environ 60 % en poids d'au moins un polymère de polyphénylsulfone (« PPSU ») , par rapport au poids total de la composition polymère ; et
(iii) moins d'environ 30 % en poids d'une charge particulaire comprenant un pigment,
dans lequel la monture de prothèse dentaire comprend au moins une ligne de finition comprenant une surface interne sensiblement plate.

2. Monture de prothèse dentaire selon la revendication 1, dans laquelle la charge particulaire a un diamètre de particules primaires moyen de 100 nm à 5 µm.

3. Monture de prothèse dentaire selon l'une quelconque des revendications 1 et 2, dans laquelle la charge particulaire comprend du Ti0₂.

4. Monture de prothèse dentaire selon l'une quelconque des revendications 1 à 3, dans laquelle la composition polymère est exempte d'une charge fibreuse.

5. Monture de prothèse dentaire selon la revendication 1, dans lequel la ligne de finition a une section transversale asymétrique dans un plan perpendiculaire à la longueur de la ligne de finition.

6. Monture de prothèse dentaire selon la revendication 6, comprenant en outre une grille de rétention comprenant au moins un trou de rétention ayant une surface inférieure à environ 10 mm².

7. Monture de prothèse dentaire selon l'une quelconque des revendications 1 à 6, comprenant en outre un support.

8. Monture de prothèse dentaire selon l'une quelconque des revendications 1 à 7, comprenant en outre un fermoir.

9. Prothèse dentaire comprenant la monture de prothèse dentaire selon l'une quelconque des revendications 6 à 8, dans laquelle la prothèse dentaire comprend une gencive artificielle disposée sur la grille de rétention et en contact avec la surface interne de la ligne de finition.

10. Prothèse dentaire selon la revendication 9, comprenant en outre au moins une dent artificielle en contact avec la gencive artificielle.

11. Procédé de formation d'une monture de prothèse dentaire selon l'une quelconque des revendications 1 à 10, le procédé comprenant :
le fraisage d'une monture de prothèse dentaire à partir d'une ébauche comprenant une composition polymère, dans lequel la composition polymère comprend :
(i) d'environ 30 % en poids à environ 80 % en poids d'au moins un polymère de poly(éther éther)cétone (« PEEK »), par rapport au poids total de la composition polymère ;
(ii) d'environ 10 % en poids à environ 60 % en poids d'au moins un polymère de polyphénylsulfone (« PPSU »), par rapport au poids total de la composition polymère ; et
(iii) moins d'environ 30 % en poids d'une charge particulaire comprenant un pigment,
et dans laquelle la monture de prothèse dentaire comprend au moins une ligne de finition comprenant une surface interne sensiblement plate.

12. Procédé selon la revendication 11, dans lequel l'ébauche comprend une ébauche cylindrique

13. Procédé selon la revendication 12, dans lequel l'ébauche cylindrique a une épaisseur d'environ 10 mm à environ 70 mm et un diamètre d'environ 20 mm à environ 100 mm.

14. Procédé selon l'une quelconque des revendications 12 et 13, comprenant en outre la fabrication de l'ébauche, dans laquelle la fabrication comprend l'extrusion de la composition polymère en une tige ayant un diamètre d'environ 20 mm à environ 100 mm et la découpe de la tige pour former l'ébauche cylindrique.
